# EUROPEAN PATENT SPECIFICATION

(11) **EP 1 673 391 B1**
(45) Date of publication and mention of the grant of the patent: **10.12.2008**
(21) Application number: 04790326.5
(22) Date of filing: 13.10.2004
(51) Int. Cl.: C07K 14/755, C12N 15/12, C12N 15/11, C12N 15/67

(54) **MODIFIED cDNA FOR HIGH EXPRESSION LEVELS OF FACTOR VIII AND ITS DERIVATIVES**
MODIFIZIERTE cDNA FÜR HOHE EXPRESSIONSNIVEAUS VON FAKTOR VIII UND DESSEN DERIVATEN
ADNc MODIFIE POUR DES NIVEAUX D'EXPRESSION ELEVES DU FACTEUR VIII ET SES DERIVES

(30) Priority: 16.10.2003 EP 03023637
(43) Date of publication of application: 28.06.2006
(73) Proprietor: Négrier, Claude, 69540 Irigny (FR)
(72) Inventor: HAUSER, Hans-Peter, 35041 Marburg (DE); PLANTIER, Jean-Luc, F-69520 Gringy (FR); DUCASSE, Cecille, F-69001 Lyon (FR); NEGRIER, Claude, F-69540 Irigny (FR)
(74) Representative: Hirsch & Associés
(86) International application number: PCT/EP2004/011445
(87) International publication number: WO 2005/040213

(56) References cited:
- EP-A- 0 260 148
- EP-A- 0 874 057
- EP-A- 1 048 726
- EP-A- 1 283 263
- PLANTIER J-L ET AL: "A FACTOR VIII MINIGENE COMPRISING THE TRUNCATED INTRON I OF FACTOR IX HIGHLY IMPROVES THE IN VITRO PRODUCTION OF FACTOR VIII" THROMBOSIS AND HAEMOSTASIS, STUTTGART, DE, vol. 2, no. 86, August 2001 (2001-08), pages 596-603, XP001068506 ISSN: 0340-6245

## Description

The present invention relates to modified DNA sequences coding for biologically active recombinant human factor VIII and its derivatives, recombinant expression vectors containing such DNA sequences, host cells transformed with such recombinant expression vectors, and processes for the manufacture of the recombinant human factor VIII and its derivatives. The invention also covers a transfer vector for use in human gene therapy which comprises such modified DNA sequences.

Classic hemophilia or hemophilia A is the most common of the inherited bleeding disorders. It results from a chromosome X-linked deficiency of blood coagulation factor VIII, and affects almost exclusively males with an incidence of between one and two individuals per 10.000. The X-chromosome defect is transmitted by female carriers who are not themselves hemophiliacs. The clinical manifestation of hemophilia A is an abnormal bleeding tendency and before treatment with factor VIII concentrates was introduced the mean life span for a person with severe hemophilia was less than 20 years. The use of concentrates of factor VIII from plasma has considerably improved the situation for the hemophilia patients. The mean life span has increased extensively, giving most of them the possibility to live a more or less normal life. However, there have been certain problems with the plasma derived concentrates and their use, the most serious of which have been the transmission of viruses. So far, viruses causing AIDS, hepatitis B, and non A non B hepatitis have hit the population seriously. Although different virus inactivation methods and new highly purified factor VIII concentrates have recently been developed no guarantees on the absence of virus contamination can be made. Also, the factor VIII concentrates are fairly expensive because the limited supply of human plasma raw material.

A factor VIII product derived from recombinant material is likely to solve a large extent of the problems associated with the use of plasma derived factor VIII concentrates for treatment for hemophilia A. However, the development of a recombinant factor VIII has met with some difficulties, for instance the problem of achieving production levels in sufficiently high yields, in particular regarding the full-length molecule.

In fresh plasma prepared in the presence of protease inhibitors, factor VIII has been shown to have a molecular weight of 280 kDa and to be composed of two polypeptide chains of 200 kDa and 80 kDa, respectively (Andersson, L.-O., et al. (1986) Proc. Natl. Aca. Sci. USA 83, 2979-2983). These chains are held together by metal ion bridges. More or less proteolytically degraded forms of the factor VIII molecule can be found as active fragments in factor VIII material purified from commercial concentrates (EP 0 197 901). The fragmented form of factor VIII having molecular weights from 260 kDa down to 170 kDa, consists of one heavy chain with a molecular weight ranging from 180 kDa down to 90 kDa, where all variants have identical amino termini, in combination with one 80 kDa light chain. The amino-terminal region of the heavy chain is identical to that of the single chain factor VIII polypeptide that can be deduced from the nucleotide sequence data of the factor VIII cDNA (Wood, W.I., et al. (1984) Nature 312, 330-336; Vehar, G.A., et al. (1984) Nature 312, 337-342).

The smallest active form of factor VIII with a molecular weight of 170 kDa, consisting of one 90 kDa and one 80 kDa chain, can be activated with thrombin to the same extent as the higher molecular weight forms, and thus represents a non activated form. It has also been shown to have full biological activity in vivo as tested in hemophilia dogs (Brinkhous, K.M., et al. (1985) Proc.Natl.Acad.Sci. USA 82, 8752-8756). Thus, the haemostatic effectiveness of the 170 kDa form is the same as for the high molecular weight forms of factor VIII.

The fact that the middle heavily glycosylated region of the factor VIII polypeptide chain residing between amino acids Arg-740 and Glu-1649 does not seem to be necessary for full biological activity has prompted several researchers to attempt to produce derivatives of recombinant factor VIII lacking this region. This has been achieved by deleting a portion of the cDNA encoding the middle heavily glycosylated region of factor VIII either entirely or partially.

For example, J.J. Toole, et al, reported the construction and expression of factor VIII, lacking amino acids 982 through 1562, and 760 through 1639 respectively (Proc.Natl.Acad.Sci. USA (1986) 83, 5939-5942). D.L. Eaton, et al. reported the construction and expression of factor VIII lacking amino acids 797 through 1562 (Biochemistry (1986) 25, 8343-8347). R.J. Kaufman described the expression of factor VIII lacking amino acids 741 through 1646 (PCT application No. WO 87/04187). N. Sarver, et al. reported the construction and expression of factor VIII lacking amino acids 747 through 1560 (DNA (1987) 6, 553-564). M. Pasek reported the construction and expression of factor VIII lacking amino acids 745 through 1562, and amino acids 741 through 1648, respectively (PCT application No. WO 88/00831). K.-D. Langner reported the construction and expression of factor VIII lacking amino acids 816 through 1598, and amino acids 741 through 1689, respectively (Behring Inst. Mitt., (1988) No. 82, 16-25, EP 295 597). P. Meulien, et al., reported the construction and expression of factor VIII lacking amino acids 868 through 1562, and amino acids 771 through 1666, respectively (Protein Engineering (1988) 2(4), 301-306, EP 0 303 540 A1). When expressing these deleted forms of factor VIII cDNA in mammalian cells the production level is typically 10 times higher as compared to full-length factor VIII.

Furthermore, attempts have been made to express the 90 kDa and 80 kDa chains separately from two different cDNA derivatives in the same cell (Burke, R.L., et al. (1986), J. Biol. Chem. 261, 12574-12578, Pavirani, A., et al. (1987) Biochem. Biophys. Res. Comm., 145, 234-240). However, in this system the in vivo reconstitution seems to be of limited efficiency in terms of recovered factor VIII:C activity.

Several studies have stressed the low factor VIII production level in different cellular systems: Biosynthesis of factor VIII was shown to be regulated in at least three different levels. First, among the factor VIII cDNA sequence two nucleotides stretchers, localized in the A2 coding domain, were demonstrated to act as transcriptional silencers (Fallaux et al., 1996; Hoeben et al., 1995; Koeberl et al., 1995; Lynch et al., 1993): Second, factor VIII protein synthesis is tightly regulated by several reticulum endoplasmic chaperones (BIP; Calreticulin; Calnexin; ERGIC-53). Many of these interactions retain factor VIII in the cell and direct it through the cellular degradation machinery (Dorner et al., 1987; Nichols et al., 1998; Pipe et al., 1998). Third, once secreted factor VIII is sensitive to protease degradation and needs to be protected by von Willebrand Factor (vWF) (Kaufman et al., 1989).

It is therefore a problem to develop improved processes which result in higher yields of factor VIII. The present invention offers a solution to this problem by a modified factor VIII cDNA.

EP1038959, EP1048726, EP EP1233064, EP1283263 and EP1284290 describe that the introduction of spliceable nucleotide sequences like a truncated FIX intron I or other introns, at positions in the cDNA which correspond to genomic factor VIII introns 1 and 13 positions can dramatically improve the expression level of factor VIII.

EP0260148 describes that introducing an intron upstream of the coding sequence of a protein like factor VIII can also increase expression levels in heterologous expression systems. EP0874057 describes a further improvement in expression level by introducing a second intron donor sequence upstream of an intron which is itself upstream of the sequence coding for the protein to be expressed like factor VIII.

This invention describes factor VIII cDNAs which result in further increases of the expression yield of factor VIII compared to prior art which can be used in an industrial process for a pharmaceutical preparation of recombinant factor VIII or its derivatives.

The invention is based on the surprising discovery that it is possible to further increase expression levels of FVIII, full-length factor as well as deletion mutants and derivatives, over EP1038959, EP1048726, EP1231220, EP1233064, EP1283263, EP1284290 and EP0260148 by combining spliceable nucleotide sequences in position 1 and 13 of the factor VIII cDNA (referring to the corresponding intron positions in the genomic FVIII clone) with a spliceable sequence upstream of they coding sequence of factor VIII.

According to the present invention such a factor VIII cDNA is modified in that at the position of intron 1 and 13 of the genomic DNA of factor VIII one or more spliceable nucleotide sequences (or a nucleotide sequence which will be spliced during the export of the pre-mRNA from the nucleus) are inserted.

Especially, if at the position of introns 1 and 13 of genomic DNA of factor VIII one or more complete or truncated introns or one or more synthetic introns which retain the ability to be spliced are inserted the level of expression of factor VIII is considerably increased as described in EP1038959, EP1048726, EP1231220, EP1233064, EP1283263 and EP1284290.

According to this invention such a factor VIII cDNA may be further modified by introducing a spliceable nucleotide sequence downstream of the promotor and upstream of the coding sequence for factor VIII.

A further object of this invention is to improve the level of expression of factor VIII and its derivatives by use of a modified factor VIII cDNA in which the B-domain of the wild-type factor VIII cDNA has been shortened or completely eliminated.

Preferably a modified factor VIII cDNA is used which comprises a first DNA segment coding for the amino acids 1 through 740 of the human factor VIII and a second DNA segment coding for the amino acids 1649 through 2332 of the human factor VIII. These two segments may be interconnected by a linker DNA segment preferably coding for a linker peptide of at least two amino acids which are selected from lysine or arginine as described in the international patent application WO 92/16557. .

The production of factor VIII proteins at high levels in suitable host cells, requires the assembly of the above-mentioned modified factor VIII DNA's into efficient transcriptional units together with suitable regulatory elements in a recombinant expression vector, that can be propagated in E. coli according to methods known to those skilled in the art. Efficient transcriptional regulatory elements could be derived from viruses having animal cells as their natural hosts or from the chromosomal DNA of animal cells. Preferably, promoter-enhancer combinations derived from the Simian Virus 40, adenovirus, BK polyoma virus, human cytomegalovirus, or the long terminal repeat of Rous sarcoma virus, or promoter-enhancer combinations including strongly constitutively transcribed genes in animal cells like beta-actin or GRP78 can be used. In order to achieve stable high levels of mRNA transcribed from the factor VIII DNA's, the transcriptional unit should contain in its 3'-proximal part a DNA region encoding a transcriptional termination-polyadenylation sequence. Preferably, this sequence is derived from the Simian Virus 40 early transcriptional region, the rabbit beta-globin gene, or the human tissue plasminogen activator gene.

The factor VIII cDNAs thus assembled into efficient recombinant expression vector are then introduced into a suitable host cell line for expression of the factor VIII proteins. Preferably this cell line should be an animal cell-line of vertebrate origin in order to ensure correct folding, disulfide bond formation, asparagines-linked glycosylation and other post-translational modifications as well as secretion into the cultivation medium. Examples on other post-translational modifications are tyrosine O-sulfation, and proteolytic processing of the nascent polypeptide chain. Examples of cell lines that can be use are monkey COS-cells, mouse L-cells, mouse C127-cells, hamster BHK-21 cells, human embryonic kidney 293 cells, and preferentially CHO-cells.

The recombinant expression vector encoding factor VIII can be introduced into an animal cell line in several different ways. For instance, recombinant expression vectors can be created from vectors based on different animal viruses, Examples of these are vectors based on baculovirus, vaccinia virus, adenovirus, and preferably bovine papilloma virus.

The transcription units encoding factor VIII can also be introduced into animal cells together with another recombinant gene, which may function as a dominant selectable marker in these cells in order to facilitate the isolation of specific cell clones, which have integrated the recombinant DNA into their genome. Examples of dominant selectable marker genes of this type are Tn5 aminoglycoside phosphotransferase, conferring resistance to Geneticin (G418), hygromycin phosphotransferase, conferring resistance to hygromycin, and puromycin acetyl transferase, conferring resistance to puromycin. The recombinant expression vector encoding such a selectable marker can reside either on the same vector as the one encoding the factor VIII cDNA, or it can be encoded on a separate vector which is simultaneously introduced and integrated to the genome of the host cell, frequently resulting in a tight physical linkage between the different transcription units.

Other types of selectable marker genes which can be used together with the factor VIII cDNAs are based on various transcription units encoding dihydrofolate reductase (dhfr). After introduction of this type of gene into cells lacking endogenous dhfr-activity, preferentially CHO-cells (DUKX-B11, DG-44) it will enable these to grow in media lacking nucleosides. An example of such a medium is Ham's F12 without hypoxanthin, thymidin, and glycine. These dhfr-genes can be introduced together with the factor VIII cDNA transcriptional units into CHO-cells of the above type, either linked on the same vector or on different vectors, thus creating dhfr-positive cell lines producing recombinant factor VIII protein.

If the above cell lines are grown in the presence of the cytotoxic dhfr-inhibitor methotrexate, new cell lines resistant to methotrexate will emerge. These cell lines may produce recombinant factor VIII protein at an increased rate due to the amplified number of linked dhfr and factor VIII transcriptional units. When propagating these cell lines in increasing concentrations of methotrexate (1-10000 nM), new cell lines can be obtained which produce factor VIII protein at very high rate.

The above cell lines producing factor VIII protein can be grown on a large scale, either in suspension culture or on various solid supports. Examples of these supports are micro carriers based on dextran or collagen matrices, or solid supports in the form of hollow fibres or various ceramic materials. When grown in suspension culture or on micro carriers the culture of the above cell lines can be performed either as a bath culture or as a perfusion culture with continuous production of conditioned medium over extended periods of time. Thus, according to the present invention, the above cell lines are well suited for the development of an industrial process for the production of recombinant factor VIII that can be isolated from human plasma.

The recombinant factor VIII protein which accumulates in the medium of CHO-cells of the above type, can be concentrated and purified by a variety of biochemical methods, including methods utilizing differences in size, charge, hydrophobicity, solubility, specific affinity, etc. between the recombinant factor VIII protein and other substances in the cell cultivation medium.

An example of such purification is the adsorption of the recombinant factor VIII protein to a monoclonal antibody, which is immobilised on a solid support. After desorption, the factor VIII protein can be further purified by a variety of chromatographic techniques based on the above properties.

The recombinant proteins with factor VIII activity described in this invention can be formulated into pharmaceutical preparations for therapeutic use. The purified factor VIII proteins may be dissolved in conventional physiologically compatible aqueous buffer solutions to which there may be added, optionally, pharmaceutical adjuvants to provide pharmaceutical preparations.

The modified factor VIII DNA's of this invention may also be integrated into a transfer vector for use in the human gene therapy.

The present invention will be further described more in detail in the following examples thereof. This description of specific embodiments of the invention will be made in conjunction with the appended drawings.

### Figures:

- Figure 1:: Expression of pD-FVIII-L2, pD-FVIII-L2-5', pD-L2-2I and pD-L2-3I in CHO cells
- Figure 2:: Expression of pD-FVIII-L2, pD-FVIII-L2-5', pD-L2-2I and pD-L2-3I in COS-7 cells
- Figure 3:: Expression of pD-FVIII-L2, pD-FVIII-L2-5', pD-L2-2I and pD-L2-3I in HKB-11 cells
- Figure 4:: Expression of pD-FVIII-L2, pD-FVIII-L2-5', pD-L2-2I and pD-L2-3I in HEK-293 cells

### Example 1: Additional effect of an Intron in the 5' extremity of the FVIII cDNA containing a truncated FIX intron I in 1 and 13 locations

### 1.1 Generation of pD-L2 vectors

All factor VIII constructs were inserted in pcDNA3.1. vector (pD) (Invitrogen, Leek, The Netherlands).

PD-FVIII-L2 (or pD-L2) and pD-FVIII-L2-I1+13 (or pD-L2-2I) were obtained by mutating a construct previously described (pD-FVIII-L0-I1+13 or pD-L0-2I) (Plantier, J. L., Rodriguez, M. H., Enjolras, N., Attali, O., and Negrier, C. (2001) Thromb Haemost 86, 596-603).

pD-L2-2I was obtained mutating pD-L0-2I using the Quickchange kit from Stratagene, following the manufacturer instructions. A portion of mutated sequence was controlled by sequencing and reintroduced in a pD-L0-2I backbone creating the pD-L2-2I vector. The oligonucleotides FVIII-L2-2I-S and FVIII-L2-2I-AS were used for mutagenesis (see Table I). The difference between the generated proteins resides in the B-domain replacing the linker which is R740-R-R-R-1649 for L0 and R740-R-R-G-G-R-R for L2.

To create a pD-L2 without intron, an Aat II restriction site was generated in pD-L0 (without intron) creating L0-new. A fragment was amplified using the oligonucleotides FVIII-N538-S and FVIII-L0-New (see Table I) and cloned in pCRII-Topo vector (InVitrogen). The Aat II site did not modify the coding sequence. Following a complete sequencing of the fragment, the Bgl II-Sal I fragment was removed and inserted in pKS-L0 opened by the same enzyme, creating pKS-L0-new. The fragment Not I-Aat II of FVIII was cleaved off and reinserted in the vector pKS-L2-2I, creating the pD-L2 without introns.

**Table I: Oligonucleotides used for creating FVIII-L2 vectors**

| **Oligos Names** | **5' to 3' Sequences** |
|---|---|
| **FVIII-L2-2I-S** | AAC AAT GCC ATT GAA CCA AGA CGT CGT GGA GGT CGA CGA GAA ATA ACT CGT |
| **FVIII-L2-2I-AS** | ACG AGT TAT TTC TCG TCG ACC TCC ACG ACG TCT TGG TTC AAT GGC ATT GTT |
| **FVIII-N538-S** | AAT ATG GAG AGA GAT CTA GCT TCA GG |
| **FVIII-L0-New-AS** | CTC GTC GAC GAC GTC TTG GTT CAA TGG |

### 1.2 Insertion of the β-globin intron 2 in 5' of the FVIII-L2 cDNA

The pSG5-plasmid containing the rabbit β-globin intron 2, was used as a matrix for PCR. The β-globin intron 2 DNA fragment was amplified, surrounded by a Nhe I and Not I sites, brought by the oligonucleotides β -Glob-Nhel-S and β -Glob-Not I-AS. The fragment was digested by both enzyme and inserted in pD-L2 or pD-L2-2I opened by the same enzymes. The fragment sequence was controlled by sequencing.

**Table II: Oligonucleotides used for β -globin cloning**

| **Oligos Names** | **Sequences** |
|---|---|
| **β -Glob-NheI-S** | CTA GCTAGC GTGAGTTTGGGGACCCTTG |
| **β -Glob-Not I-AS** | ATAGTTTA GCGGCCGC TGTAGGAAAAAGAAGAAGGC |

### 1.3 Cell culture conditions

CHO and COS-1 cells were obtained from ECACC (Sigma, L'Isle d'Abeau, France). HEK-293 and HKB-11 cells were obtained from LGC Promochem (Molsheim, France), the ATCC French distributor. All the cell culture reagents (media, antibiotics, supplements and serum) are from InVitrogen (Cergy Pontoise, France). Cells were incubated at 37°C in a 5% CO₂ humidified incubator. CHO and COS-1 cells were grown in IMDM medium supplemented with 10% fetal bovine serum, 2 mM L-Glutamine and 1% penistreptomycin. HEK-293 cells were grown in EMEM medium supplemented with 10% fetal bovine serum, 1% NEAA, 2mM L-Glutamine and 1% penistreptomycin. HKB-11 cells were grown in RPMI medium supplemented with 2,5% fetal bovine serum, 2% HAT, 2mM L-Glutamine and 1% penistreptomycin.

### 1.4 Transfection conditions

5.10⁵ cells (or 1x 10⁶ for HEK-293) were plated in a 9.5 cm² dish. One day later, cells were incubated for 6 hours with 1µg of DNA pre-complexed with 5µl FuGENE-6 reagent, following the manufacturer recommendations. Then, new medium was added.

48 hours after transfection, cell dishes were washed 3 times with PBS. The cells were then incubated for 6 hours in SVF-free medium containing 1% BSA.

### 1.5 Factor VIII expression analysis

FVIII antigen concentrations were measured in the cell conditioned media using an ELISA kit (Asserachrom FVIII, Stago, Asnières, France).

The activity was controlled using the Coamatic kit (Chromogenix, Milano, Italia) following the recommendations of the manufacturer. The activity was measured in a range 2-8 ng/ml. Two dilutions were analyzed for each sample.

### 1.6 Results

The following constructs were transfected using FuGENE-6 in different cell lines: pD-FVIII-L2 (L2, without intron), pD-FVIII-L2-5' (5'I, with the β-globin intron 2 in 5'), pD-L2-2I (2I, with the TFIXI1 in 1 and 13 locations) and pD-L2-3I (3I, with a β - globin intron 2 in 5'and the TFIXI1 in 1 and 13 locations). As negative controls, non-transfected cells were identically treated. Two days following transfection, the cells were incubated for 6 h in IMDM containing 1% BSA. The quantity of FVIII produced during this period was quantified using an ELISA.

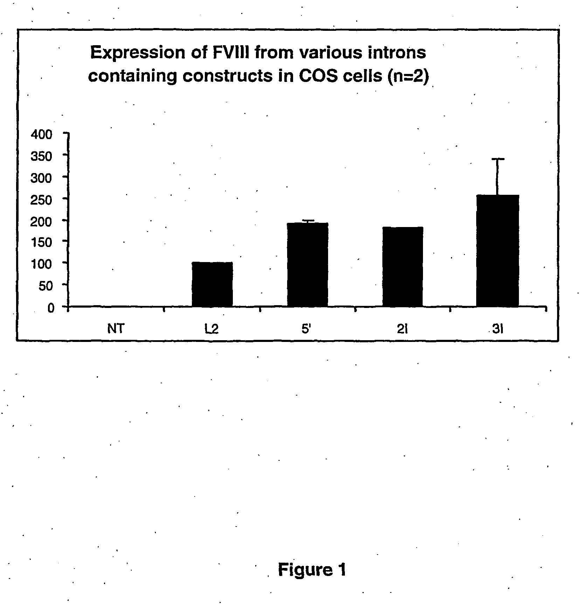

Two independent transfections were done in CHO cells (figure 1). The ratio of FVIII produced from the construct without intron was used as the basis level. The results are presented as a percentage of increase in FVIII production compared to this basis level.

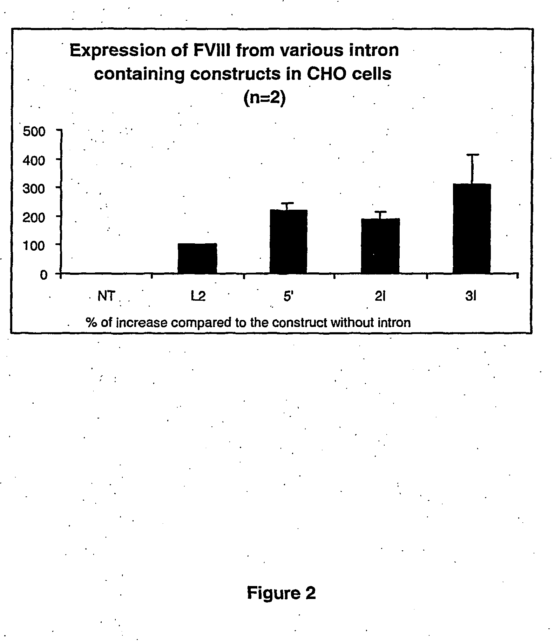

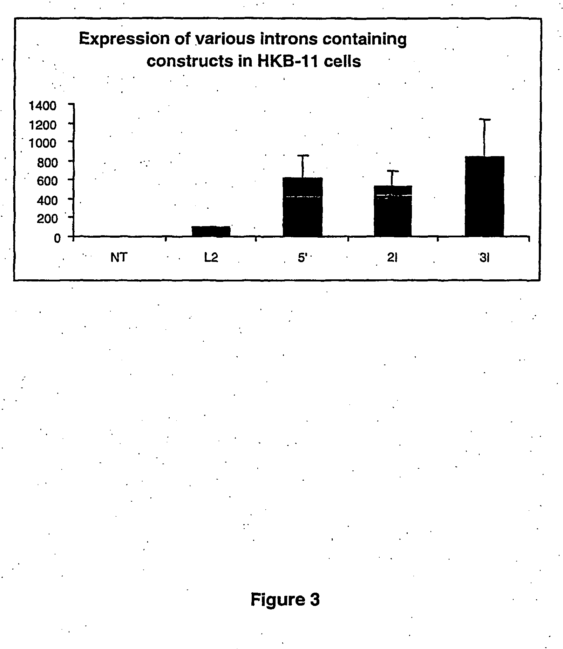

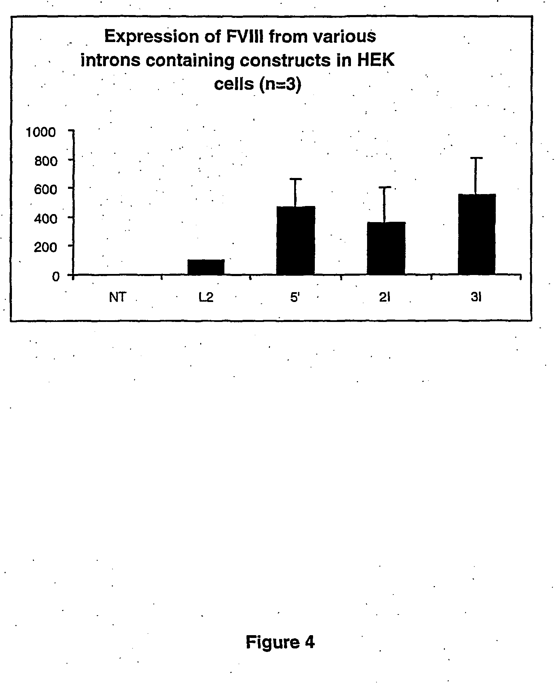

An identical experiment was repeated in COS-7 cells (figure 2) in HKB-11 cells (figure 3) and HEK-293 cells (figure 4), except that for the two later sets of results three transfections were made.

A summary of the increase in FVIII production compared to the construct devoid of introns is presented in the following table. The results from the four cell lines assayed in shown.

**Table 3: Summary of the obtained values**

| | **CHO** | **COS-7** | **HEK-293** | **HKB-11** |
|---|---|---|---|---|
| **FVIII-L2** | 100 | 100 | 100 | 100 |
| **FVIII-L2-5'** | 220 | 191 | 471 | 624 |
| **FVIII-L2-2I** | 187 | 180 | 357 | 524 |
| **FVIII-L2-3I** | 312 | 255 | 555 | 837 |

All constructs containing introns lead in all cell lines to significantly higher expression of factor VIII than the construct without. The construct with either two introns within the FVIII sequence or a unique intron in the 5' position gave similar levels of production with however some better expression level for clones with the 5' intron.

In contrast the 3 intron construct (containing a 5' intron and introns at intron positions 1 and 13 of the factor VIII genomic DNA) was the construct that consistently lead to highest expression levels in all cell lines. The increase in expression yield was depending on the cell line 20-42% as compared to the 5' intron clone or 40-67% as compared to the 1+13 intron clone. Therefore this construct will be of interest to improve current technology to express FVIII.

## Claims

1. Modified factor VIII cDNA, **characterized in that** in positions where introns 1 and 13 in the genomic factor VIII sequence are inserted, the cDNA of factor VIII also contains one or more spliceable nucleotide sequences or a nucleotide sequence which will be spliced during the export of the pre-mRNA from the nucleus and in addition another spliceable nucleotide sequence which is inserted downstream of the promoter sequence and upstream of the modified factor VIII cDNA.

2. Modified factor VIII cDNA as claimed in claim 1, **characterized in that** in introns positions 1 and/or 13 of the genomic factor VIII sequence one or more complete or truncated introns have been inserted.

3. Modified factor VIII cDNA as claimed in claim 1, **characterized in that** in intron positions 1 and/or 13 of the genomic factor VIII sequence one or more natural occurring or synthetic nucleic acid sequences, which retain the ability to be spliced have been inserted.

4. Modified factor VIII cDNA as claimed in claim 1, **characterized in that** in intron positions 1 and 13 of the genomic factor VIII sequence a truncated FIX introns I has been inserted.

5. Modified factor VIII cDNA as claimed in claim 1, **characterized in that** downstream of the promoter and upstream of the FVIII coding sequence one complete or truncated intron has been inserted.

6. Modified factor VIII cDNA as claimed in claim 1, **characterized in that** downstream of the promoter and upstream of the FVIII coding sequence one natural occurring or synthetic nucleic acid sequences which retain the ability to be spliced have been inserted.

7. Modified factor VIII cDNA as claimed in claim 1, **characterized in that** downstream of the promoter and upstream of the FVIII coding sequence a β-globin intron 2 has been inserted.

8. Modified factor VIII cDNA as claimed in claims 1 or 4, **characterized in that** it comprises a first DNA segment coding for the amino acids 1 through 740 of the human factor VIII and a second DNA segment coding for the amino acids 1649 through 2332 of the human factor VIII, said segments being interconnected by a linker DNA segment coding for a linker peptide of at least two amino acids which are selected from lysine and arginine.

9. Recombinant expression vector containing a transcription unit comprising the modified factor VIII cDNA sequence according to claims 1 to 5, a transcriptional promoter and a polyadenylation sequence.

10. A host cell line of animal origin transformed with the recombinant expression vector of claim 9, further **characterized in that** said host cell line is not a human embryonic stem cell line.

11. Process for the production of a biologically active recombinant human factor VIII or its derivative, **characterized in that** the production is performed by cultivating the animal cell line of claim 10 in a nutrient medium allowing expression and secretion of the human factor VIII or its derivative and recovering said expression product from the culture medium.

12. Transfer vector for use in the human gene therapy, **characterized in that** it comprises a modified factor VIII cDNA as claimed in claims 1 to 5.

13. A host cell according to claim 10, **characterized in that** it is a human cell grown in suspension culture or on solid support.

## Patentansprüche

1. Modifizierte Faktor VIII-cDNA, **dadurch gekennzeichnet, dass** in Positionen, wo Intronen 1 und 13 in der genomischen Faktor VIII-Sequenz eingefügt sind, die cDNA von Faktor VIII auch eine oder mehrere spleißbare Nucleotidsequenzen oder eine Nucleotidsequenz enthält, die während des Exports der Prä-mRNA von dem Nucleus gespleißt wird, und zusätzlich eine andere spleißbare Nucleotidsequenz, die stromabwärts der Promotor-Sequenz und stromaufwärts der modifizierten Faktor VIII-cDNA eingesetzt ist.

2. Modifizierte Faktor VIII-cDNA nach Anspruch 1, **dadurch gekennzeichnet, dass** in den Intron-Positionen 1 und/oder 13 der genomischen Faktor VIII-Sequenz ein oder mehrere komplette oder verkürzte Intronen eingesetzt sind.

3. Modifizierte Faktor VIII-cDNA nach Anspruch 1, **dadurch gekennzeichnet, dass** in den Intron-Positionen 1 und/oder 13 der genomischen Faktor VIII-Sequenz eine oder mehrere natürlich auftretende oder synthetische Nucleinsäuresequenzen, die die Fähigkeit, gespleißt zu werden, behalten, eingesetzt sind.

4. Modifizierte Faktor VIII-cDNA nach Anspruch 1, **dadurch gekennzeichnet, dass** in den Intron-Positionen 1 und 13 der genomischen Faktor VIII-Sequenz ein verkürztes FIX-Intron I eingesetzt ist.

5. Modifizierte Faktor VIII-cDNA nach Anspruch 1, **dadurch gekennzeichnet, dass** stromabwärts des Promotors und stromaufwärts der FVIII-Codierungssequenz ein komplettes oder verkürztes Intron eingesetzt ist.

6. Modifizierte Faktor VIII-cDNA nach Anspruch 1, **dadurch gekennzeichnet, dass** stromabwärts des Promotors und stromaufwärts der FVIII-Codierungssequenz eine natürlich auftretende oder eine synthetische Nucleinsäuresequenz, die die Fähigkeit, gespleißt zu werden, behält, eingesetzt ist.

7. Modifizierte Faktor VIII-cDNA nach Anspruch 1, **dadurch gekennzeichnet, dass** stromabwärts des Promotors und stromaufwärts der FVIII-Codierungssequenz ein β-Globin-Intron 2 eingesetzt ist.

8. Modifizierte Faktor VIII-cDNA nach Anspruch 1 oder 4, **dadurch gekennzeichnet, dass** sie ein erstes DNA-Segment umfasst, das für die Aminosäuren 1 bis 740 des humanen Faktor VIII codiert, und ein zweites DNA-Segment, das für die Aminosäuren 1649 bis 2332 des humanen Faktor VIII codiert, wobei die Segmente durch ein Linker-DNA-Segment verbunden sind, das für ein Linker-Peptid aus mindestens zwei Aminosäuren codiert, die aus Lysin und Arginin ausgewählt sind.

9. Rekombinanter Expressionsvektor, der eine Transkriptionseinheit enthält, die die modifizierte Faktor VIII-cDNA-Sequenz nach einem der Ansprüche 1 bis 5, einen Transkriptions-Promotor und eine Polyadenylierungssequenz enthält.

10. Wirtszelllinie tierischen Ursprungs, die mit dem rekombinanten Expressionsvektor nach Anspruch 9 transformiert ist, des Weiteren **dadurch gekennzeichnet, dass** die Wirtszelllinie keine humane embryonische Stammzelllinie ist.

11. Verfahren für die Herstellung eines biologisch aktiven, rekombinanten, humanen Faktor VIII oder dessen Derivates, **dadurch gekennzeichnet, dass** die Herstellung durch Kultivieren der tierischen Zelllinie von Anspruch 10 in einem Nährmedium, das eine Expression und Sekretion des humanen Faktor VIII oder seines Derivates ermöglicht, und Gewinnen des Expressionsprodukts aus dem Kulturmedium durchgeführt wird.

12. Transfervektor zur Verwendung in der Human-Gentherapie, **dadurch gekennzeichnet, dass** er eine modifizierte Faktor VIII-cDNA nach einem der Ansprüche 1 bis 5 umfasst.

13. Wirtszelle nach Anspruch 10, **dadurch gekennzeichnet, dass** sie eine humane Zelle ist, die in Suspensionskultur oder auf einem festen Träger gezüchtet wird.

## Revendications

1. ADNc modifié codant pour le Facteur VIII **caractérisé en ce que** dans les positions où les introns 1 et 13 sont insérés dans la séquence génomique du facteur VIII, l'ADNc codant pour le Facteur VIII contient également une ou plusieurs séquences nucléotidiques susceptibles d'être épissées ou une séquence nucléotidique qui sera épissée durant l'export du pré-ARNm depuis le noyau et en outre une autre séquence nucléotidique susceptible d'être épissée qui est insérée en amont de la séquence promoteur et en aval de l'ADNc modifié codant pour le Facteur VIII.

2. ADNc modifié codant pour le Facteur VIII tel que revendiqué dans la revendication 1, **caractérisé en ce que** dans les positions 1 et/ou 13 des introns de la séquence génomique du facteur VIII ont été insérés un ou plusieurs introns complets ou tronqués.

3. ADNc modifié codant pour le Facteur VIII tel que revendiqué dans la revendication 1, **caractérisé en ce que** dans les positions 1 et/ou 13 des introns de la séquence génomique du facteur VIII ont été insérées une ou plusieurs séquences d'acides nucléiques produites naturellement ou produites par synthèse, qui conservent leur aptitude à être épissées.

4. ADNc modifié codant pour le Facteur VIII tel que revendiqué dans la revendication 1, **caractérisé en ce que** dans les positions 1 et/ou 13 des introns de la séquence génomique du facteur a été inséré un intron 1 de facteur IX tronqué.

5. ADNc modifié codant pour le Facteur VIII tel que revendiqué dans la revendication 1, **caractérisé en ce que** en aval du promoteur et en amont de la séquence codant pour le facteur VIII ont été insérés un ou plusieurs introns complets ou tronqués.

6. ADNc modifié codant pour le Facteur VIII tel que revendiqué dans la revendication 1, **caractérisé en ce que** en aval du promoteur et en amont de la séquence codant pour le facteur VIII ont été insérées une ou plusieurs séquences d'acides nucléiques produites naturellement ou produites par synthèse, qui conservent leur aptitude à être épissées.

7. ADNc modifié codant pour le Facteur VIII tel que revendiqué dans la revendication 1, **caractérisé en ce que** en aval du promoteur et en amont de la séquence codant pour le facteur VIII a été inséré un intron 2 de β-globine.

8. ADNc modifié codant pour le Facteur VIII tel que revendiqué dans les revendications 1 ou 4, **caractérisé en ce qu'**il comprend un premier segment d'ADN codant pour les acides aminés 1 à 740 du facteur VIII humain et un second segment d'ADN codant pour les acides aminés 1649 à 2332 du facteur VIII humain, lesdits segments étant interconnectés par un segment d'ADN lieur codant pour un peptide lieur d'au moins deux acides aminés choisis parmi la lysine et l'arginine.

9. Vecteur d'expression recombinant contenant un élément de transcription comprenant la séquence d'ADNc modifié codant pour le facteur VIII selon les revendications 1 à 5, un promoteur transcriptionnel et une séquence de polyadénylation.

10. Une lignée de cellules hôtes d'origine animale transformées par le vecteur d'expression recombinant de la revendication 9, **caractérisé en outre en ce que** ladite lignée de cellules hôtes n'est pas un lignée de cellules souches embryonnaires humaine.

11. Procédé de production d'un facteur VIII recombinant humain biologiquement actif ou d'un de ses dérivés, **caractérisé en ce que** la production est réalisée par culture de la lignée de cellules animales de la revendication 10 dans un milieu nutritif permettant l'expression et la sécrétion du facteur VIII humain ou de ses dérivés et la récupération dudit produit d'expression du milieu de culture.

12. Vecteur de transfert pour son utilisation en thérapie génique humaine, **caractérisé en ce qu'**il comprend ADNc modifié codant pour le facteur VIII tel que revendiqué dans les revendications 1 à 5.

13. Une cellule hôte selon la revendication 10, **caractérisée en ce qu'**elle consiste en une cellule humaine développée dans une culture en suspension ou sur un support solide.


### SEQUENCE LISTING

<110> ZLB Behring GmbH
<120> Modified cDNA for high expression levels of factor VIII and its derivatives
<130> 2003/M016[A077]
<150> 03023637.6
   <151> 2003-10-16
<160> 8
<170> PatentIn version 3.1
<210> 1
   <211> 51
   <212> DNA
   <213> Artificial Sequence
<220>
   <223> FVIII-L2-21-S
<400> 1
   aacaatgcca ttgaaccaag acgtcgtgga ggtcgacgag aaataactcg t 51
<210> 2
   <211> 51
   <212> DNA
   <213> Artificial Sequence
<220>
   <223> FVIII-L2-21-AS
<400> 2
   acgagttatt tctcgtcgac ctccacgacg tcttggttca atggcattgt t 51
<210> 3
   <211> 26
   <212> DNA
   <213> Artificial sequence
<220>
   <223> FVIII-N538-S
<400> 3
   aatatggaga gagatctagc ttcagg 26
<210> 4
   <211> 27
   <212> DNA
   <213> Artificial sequence
<220>
   <223> FVIII-L0-New-AS
<400> 4
   ctcgtcgacg acgtcttggt tcaatgg 27
<210> 5
   <211> 28
   <212> DNA
   <213> Artificial sequence
<220>
   <223> b-Glob-Nhel-s
<400> 5
   ctagctagcg tgagtttggg gacccttg 28
<210> 6
   <211> 36
   <212> DNA
   <2l3> Artificial Sequence
<220>
   <223> b-Glob-Not I-AS
<400> 6
   atagtttagc ggccgctgta ggaaaaagaa gaaggc 36
<210> 7
   <211> 30
   <212> DNA
   <213> Artificial Sequence
<220>
   <223> F8/AG-kozak.s
<400> 7
   cggcttacac ccatgcaaat agagctctcc 30
<210> 8
   <211> 30
   <212> DNA
   <213> Artificial sequence
<220>
   <223> F8/ATG-kozal.AS
<400> 8
   ggagagctct atttgcatgg gtgtaagccg 30